**Europäisches Patentamt**

**European Patent Office**

**Office européen des brevets**

(19)

(11) Veröffentlichungsnummer: **0 194 488**

**A2**

# EUROPÄISCHE PATENTANMELDUNG

(12)

(21) Anmeldenummer: **86102296.0**

(22) Anmeldetag: **21.02.86**

(51) Int. Cl.⁴: **A61B 8/08** , A01K 29/00

(30) Priorität: **15.03.85 DE 8507700 U**

(43) Veröffentlichungstag der Anmeldung:
**17.09.86 Patentblatt 86/38**

(84) Benannte Vertragsstaaten:
**AT BE CH DE FR GB IT LI LU NL SE**

(71) Anmelder: **Rheintechnik Weiland & Kaspar KG
Grube König
D-6680 Neunkirchen/Saar(DE)**

(72) Erfinder: **Weiland, Werner
Koblenz-Olper-Strasse 172
D-5413 Bendorf-Sayn(DE)**

(74) Vertreter: **Röbe-Oltmanns, Georg, Dr.
Dotzheimer Strasse 61
D-6200 Wiesbaden(DE)**

(54) **Gerät zur Feststellung der Trächtigkeit von weiblichen Haustieren.**

(57) Die Erfindung betrifft ein Gerät zur Feststellung der Trächtigkeit von weiblichen Haustieren, insbesondere Schweinen, Schafen, Ziegen und Kühen bestehend aus einem Ultraschallsender und -empfänger, einem Gehäuse, in dem ein Signalgeber und eine Batterie angeordnet sind, wobei Sender und Empfänger einerseits sowie Signalgeber und Batterie andererseits durch ein Kabel miteinander verbunden sind. Eine erste Ader des Kabels ist auf der einer Koppelschicht zugekehrten Seite mit einer Piezooxidscheibe und eine zweite Ader des Kabels, auf der einer Koppelschicht abgekehrten Seite mit einer Piezooxidscheibe verbunden und im Gehäuse ist eine Platine angeordnet, die eine Durchbrechung aufweist, deren Rand den Rand des Signalgeberkorbes übergreift und durch die Durchbrechung der Platine der Signalgebermagnet ragt.

FIG. 1

EP 0 194 488 A2

Gerät zur Feststellung der Trächtigkeit von weiblichen Haustieren

Die Neuerung betrifft ein Gerät zur Feststellung der Trächtigkeit von weiblichen Haustieren, insbesondere Schweinen, Schafen, Ziegen und Kühen, bestehend aus einem Ultraschallsender und -empfänger, einem Gehäuse, in dem ein Signalgeber und eine Batterie angeordnet sind, wobei Sender und Empfänger einerseits sowie Signalgeber und Batterie anderseits durch ein Kabel miteinander verbunden sind.

Geräte dieser Art sind bekannt. Sie dienen zur Feststellung der Trächtigkeit von weiblichen Haustieren, insbesondere Schweinen, Schafen, Ziegen und Kühen. Der Ultraschallsender und -empfänger wird von außen auf den Körper des Haustieres aufgesetzt. Die vom Ultraschallsender ausgesandten Ultraschallwellen werden vom Embryo in der Gebärmutter reflektiert, sobald das Embryo eine ausreichende Größe hat, und die reflektierten Wellen werden dann vom Empfänger aufgenommen. Die reflektierten Wellen werden durch entsprechende elektrische Schalt- und Steuerelemente in ein akustisches oder optisches Signal umgesetzt, das dann vom Bauern von außen an einem entsprechenden Signalgeber am Gerät wahrgenommen wird.

Die bekannten Geräte dieser Art haben den Nachteil, daß sie relativ hoch, breit und lang sind.

Aufgabe vorliegender Erfindung ist es, ein Gerät der eingangs genannten Art zu schaffen, welches schmäler, niedriger und kürzer ist.

Diese Aufgabe wird bei einem Gerät der eingangs genannten Art dadurch gelöst, daß der Ultraschallsender und -empfänger aus einer Piezooxidscheibe, die in einem Ende eines Rohrstückes angeordnet ist, wobei zwischen Piezooxidscheibe und dem freien Ende des Rohrstückes eine Koppelschicht angeordnet ist, auf der der Koppelschicht abgekehrten Seite der Piezooxidscheibe eine Füllung in das Rohrstück eingebracht ist, auf der der Koppelschicht abgekehrten Seite das Kabel durch die Füllung bis an die Piezooxidscheibe geführt ist und eine erste Ader des Kabels auf der der Koppelschicht zugekehrten Seite mit der Piezooxidscheibe sowie eine zweite Ader des Kabels auf der der Koppelschicht abgekehrten Seite mit der Piezooxidscheibe verbunden ist, wobei im Gehäuse eine Platine angeordnet ist, die Platine eine Durchbrechung aufweist, der Rand der Durchbrechung den Rand des Signalgeberkorbes übergreift und durch die Durchbrechung der Platine der Signalgebermagnet ragt.

Der Rand der Piezooxidscheibe sollte eine Einkerbung für die andere Ader des Kabels aufweisen.

Um so weit wie möglich im rauhen Stallbetrieb Beschädigungen des Ultraschallsenders und -empfängers zu vermeiden, ist das freie Ende des Rohrstückes vorzugsweise mit einem Gummiring versehen. Das Kabel ist zweckmäßig ein Koaxialkabel.

Durch die eine Seitenwand des Gehäuses ragt der Schaltstift eines auf der Innenseite des Gehäuses angeordneten Ein-und Ausschalters. Der Signalgeber ist zweckmäßig ein Lautsprecher. Die Platine sollte auf der dem Signalgeberkorb abgekehrten Seite elektrische Schalt-, Speicher-und Steuerelemente aufweisen, die einen Impulsgeber, Impulsverstärker, Verstärker, Schwellwertschalter, Speicher-Flip-Flop, Leistungsendstufe usw. enthalten.

Das Gehäuse weist auf der Rückseite zweckmäßig einen Klipp auf, mit dem der Benutzer das Gerät, insbesondere das Gehäuse, an seiner Kleidung befestigen kann, z.B. am Hosenbund.

In den Figuren ist die Erfindung an einer Ausführungsform beispielsweise dargestellt, ohne auf diese Ausführungsform beschränkt zu sein.

Es stellt dar:

Figur 1 einen Schnitt durch das erfindungsgemäße Gerät,

Figur 2 ein Blockschaltbild des Gerätes.

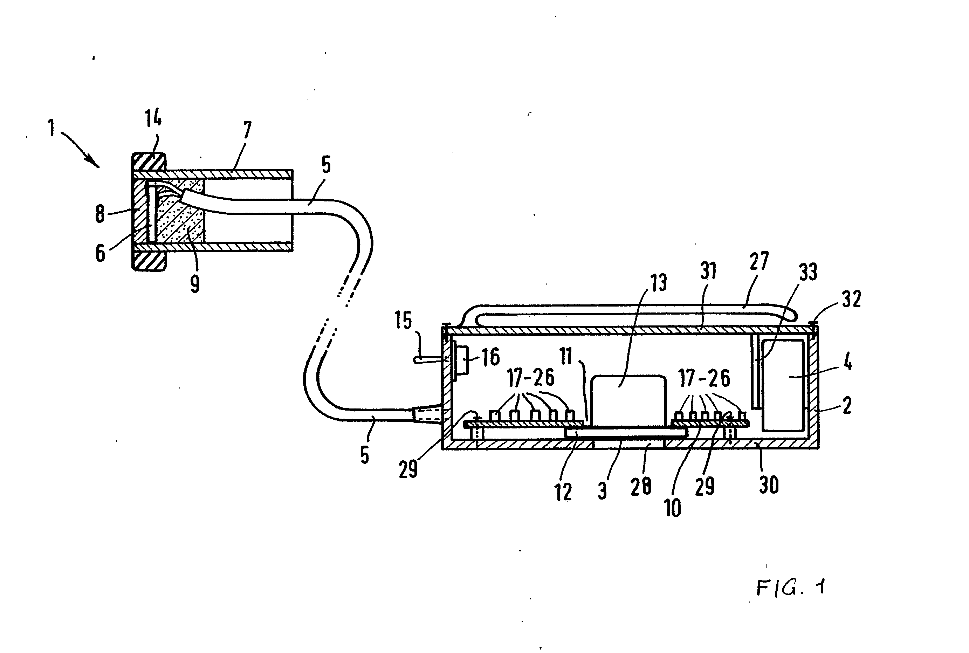

Das in Fig. 1 dargestellte Gerät besteht aus dem Ultraschallsender und -empfänger, der aus einem Rohrstück 7 besteht, in dessen einem Ende in Abstand vom freien Ende eine Piezooxidscheibe 6 angeordnet ist. Zwischen der Piezooxidscheibe 6 und dem freien Ende des Rohrstücks 7 befindet sich ein Koppelstück 8. Auf der dem Koppelstück 8 abgekehrten Seite der Piezooxidscheibe 6 ist eine Füllung 9 vorgesehen. Durch die Füllung 9 ist das Kabel 5 mit dem einen Ende geführt. Die eine Ader des Kabels 5 ist auf der der Koppelschicht 8 zugekehrten Seite mit der Piezooxidscheibe verbunden und die andere Ader auf der anderen Seite. Seitlich weist die Piezooxidscheibe 6 eine Einkerbung für die eine Ader des Kabels 5 auf. Das freie Ende des Rohrstücks 7 ist mit einem Gummiring 14 versehen, um Beschädigungen des Ultraschallsenders und -empfängers so weit wie möglich zu vermeiden. Das andere Ende des Kabels 5 führt zu dem Gehäuse 2, in dem der Signalgeber 3, vorzugsweise ein Lautsprecher, und die Batterie 4 angeordnet sind. Durch die Seitenwand des Gehäuses 2, durch die das Kabel 5 geführt ist, ragt der Schaltstift 15 eines Ein-Ausschalters 16, der auf der Innenseite dieser Seitenwand des Gehäuses befestigt ist. Im Gehäuse 2 ist unten eine Platine 10 angeordnet, die eine Durchbrechung 11 aufweist. Der Rand der Durchbrechung 11 übergreift den Rand des Signalgeberkorbes 12. Durch die Durchbrechung 11 ragt der Signalgebermagnet 13 in den Innenrahm des Gehäuses. Auf der Austtritsseite des Signalgebers 3 ist im Gehäuse 2 eine Durchbrechung 28 vorgesehen. Die Platine 10 ist mit Stiftschrauben 29 oder dergleichen am Boden 30 des Gehäuses befestigt. Auf der dem Boden 30 abgekehrten Seite trägt die Platine 10 elektrische Schalt-, Speicher-und Steuerelemente 17 bis 26, wie einen Impulsgeber, Impulsverstärker, Verstärker, Schwellwertschalter, Speicher-Flip-flop, Leistungsendstufe und dergleichen.

Das Gehäuse 2 ist oben mit dem Deckel 31 verschlossen, der mit Stiftschrauben 32 oder dergleichen am Gehäuse 2 befestigt ist. Auf der Innenseite weist der Deckel 31 einen Steg 33 auf, der den Raum für die Batterie 4 von Raum für die Platine 10 und den Signalgeber 3 trennt. Die beiden Adern des Kabels 5 führen vom Kabel 5 durch den Ein-Ausschalter 16 zwischen Boden 30 und Platine 10 hindurch zur Batterie 4. Der Schwellwertschalter dient dem Zwecke sicherzustellen, daß nur reflektierte Wellen entsprechender Stärke in ein Signal umgesetzt werden. Im Speicher-Flip-flop wird das Signal für einen Zeitraum festgehalten, der sicherstellt, daß der Signalgeber anspricht.

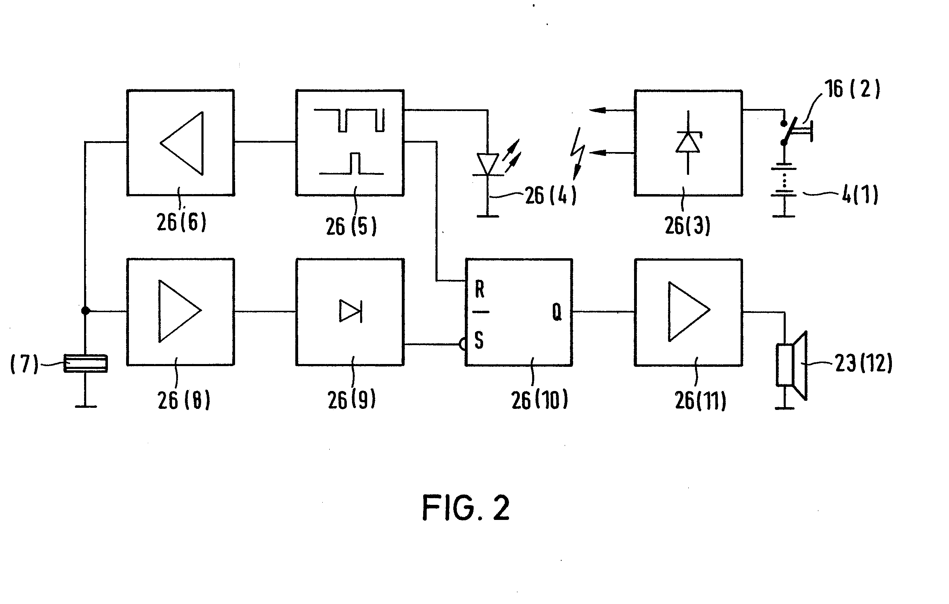

Das in dem in Figur 2 dargestellten Blockschaltbild bedeutet:

(1) 9-Volt-Batterie

(2) Ein-/Aus-Schalter

(3) Stabilisierung und Hochspannungserzeugung

(4) Betreibsbereitschaftsanzeige

(5) 3 Impulsgeneratoren für:

a) Sendeimpulse

b) Kontakt-/Trächtigkeitsfenster

c) Umschaltung Kontakt-/Trächtigkeitsanzeige

(6) Sendeimpulsverstärker

(7) Ultraschallsonde

(8) Empfangsimpulsverstärker

(9) Gleichrichter und Schwellwertschalter

(10) R/S-Flip-Flop mit R-Priorität

(11) Leistungsverstärker für Lautsprecher

(12) Dynamischer Kleinlautsprecher

**Ansprüche**

1.) Gerät zur Feststellung der Trächtigkeit von weiblichen Haustieren, insbesondere Schweinen, Schafen, Ziegen und Kühen, bestehend aus einem Ultraschallsender und -empfänger (1), einem Gehäuse (2), in dem ein Signalgeber (3) und eine Batterie (4) angeordnet sind, wobei Sender/Empfänger (1) einerseits sowie Signalgeber (3) und Batterie (4) andererseits durch ein Kabel (5) miteinander verbunden sind,

gekennzeichnet durch

einen Ultraschallsender und -empfänger (1) aus einer Piezooxidscheibe (6), die in einem Ende eines Rohrstückes (7) angeordnet ist, wobei zwischen Piezooxidscheibe (6) und dem freien Ende des Rohrstückes (7) eine Koppelschicht (8) angeordnet ist, auf der der Koppelschicht (8) abgekehrten Seite der Piezooxidscheibe (6) eine Füllung (9) in das Rohrstück (7) eingebracht ist, auf der der Koppelschicht (8) abgekehrten Seite das Kabel (5) durch die Füllung (9) bis an die Piezooxidscheibe (6) geführt ist und eine erste Ader des Kabels (5) auf der der Koppelschicht (8) zugekehrten Seite mit der Piezooxidscheibe (6) sowie eine zweite Ader des Kabels (5) auf der der Koppelschicht (8) abgekehrten Seite mit der Piezooxidscheibe verbunden ist und im Gehäuse (2) eine Platine (10) angeordnet ist, die Platine (10) eine Durchbrechung (11) aufweist, der Rand der Durchbrechung (11) den Rand des Signalgeberkorbes (12) übergreift und durch die Durchbrechung (11) der Platine (10) der Signalgebermagnet (13) ragt.

2.) Gerät nach Anspruch 1,

dadurch gekennzeichnet, daß das Rohrstück (7) aus einem Kunststoff besteht.

3.) Gerät nach Anspruch 1,

dadurch gekennzeichnet, daß das Rand der Piezooxidscheibe (6) eine Einkerbung für die eine Ader des Kabels (5) aufweist.

4.) Gerät nach einem der Ansprüche 1 bis 3,

dadurch gekennzeichnet, daß das freie Ende des Rohrstücks (7) einen Gummiring (14) trägt.

5.) Gerät nach einem der Ansprüche 1 bis 4,

dadurch gekennzeichnet, daß das Kabel (5) ein Koaxialkabel ist.

6.) Gerät nach einem der Ansprüche 1 bis 5,

dadurch gekennzeichnet, daß durch eine Seitenwand des Gehäuses (2) der Schaltstift (15) eines auf der Innenseite des Gehäuses angeordneten Ein-Ausschalters (16) ragt.

7.) Gerät nach einem oder mehreren der Ansprüche 1 bis 6,

dadurch gekennzeichnet, daß der Signalgeber (3) ein Lautsprecher ist.

8.) Gerät nach einem oder mehreren der Ansprüche 1 bis 7,

dadurch gekennzeichnet, daß die Platine (10) auf der dem Signalgeberkorb (12) abgekehrten Seite elektrische Schalt-, Speicher- und Steuerelemente (16 bis 26), wie einen Impulsgeber, Impulsverstärker, Verstärker, Stellwertschalter, Speicherflip-flop, Leistungsendstufe und dergleichen, trägt.

9.) Gerät nach einem oder mehreren der Ansprüche 1 bis 8,

dadurch gekennzeichnet, daß das Gehäuse (2) auf der Rückseite einen Klipp (27) aufweist.

FIG. 1

FIG. 2

0 194 488